# EUROPEAN PATENT APPLICATION

(11) **EP 1 891 899 A1**
(43) Date of publication of application: **27.02.2008**
(21) Application number: 07016735.8
(22) Date of filing: 27.08.2007
(51) Int. Cl.: A61B 6/03

(54) **Method and system for performing local tomography**

(30) Priority: 25.08.2006 US 840010 P
(71) Applicant: Multi Magnetics Incorporated, London ON (CA)
(72) Inventor: Stodilka, Robert Z., London Ontario N5V 2K2 (CA); Prato, Frank S., London Ontario N5Y 1P2 (CA)
(74) Representative: Naismith, Robert Stewart

(57) **Abstract**

A system and method for performing local tomography is provided. The system comprises an anatomical imaging subsystem for imaging a patient and generating anatomical image data. A computing device generates an anatomical image from the anatomical image data and identifies a region-of-interest within the anatomical image. A molecular imaging subsystem captures molecular image data from only a portion of the patient including the region-of-interest. The computing device generates a molecular tomograpic image of the region-of-interest from the molecular image data and the anatomical image.

## Description

### Field of the Invention

The present invention relates generally to medical imaging and, in particular, to a method and system for performing local tomography.

### Background of the Invention

Medical imaging is a rapidly growing field, and includes many imaging modalities, each facilitating the visualization of different aspects of the body and disease processes, and each possessing its own strengths and weaknesses. Medical imaging can generally be divided into two categories: anatomical imaging and molecular imaging. Anatomical imaging allows for the visualization of endogenous contrast, and provides for the imaging of soft-tissues and hard-tissues. Anatomical imaging is well-established and types include X-ray computed tomography ("X-ray CT" or, more simply, "CT") and magnetic resonance imaging ("MRI").

Molecular imaging involves the non-invasive measurement of the distribution of specific biological processes in the body that can act as indicators for abnormalities such as disease. Molecular imaging, which visualizes molecular processes using exogenous contrast, is often compromised by its inability to visualize anatomical information.

As will be appreciated, it is of interest to detect biological processes early in the disease process - hopefully before the disease manifests anatomical distortions.
Such early detection is challenging because medical imaging technology is often insensitive to subtle changes in biochemistry and metabolism. These subtle changes often do not create enough endogenous contrast to be detected without aid. Using molecular imaging, this aid comes in the form of artificial contrast agents that are introduced into the body (that is, exogenous agents) for the purpose of highlighting subtle changes at the biochemical and metabolic level. In general, these exogenous contrast agents have two useful properties: they distribute in the body according to a known pattern that is indicative of health or disease, and they are easily measurable by a detector external to the body. One example of an exogenous contrast agent is Tc-99m-Sestamibi, which becomes distributed according to cardiac perfusion. Its radionuclide, Tc-99m, decays and emits a gamma ray that is detectable by a gamma-ray detector.

Two common molecular imaging types are single photon emission computed tomography ("SPECT") and positron emission tomography ("PET"). SPECT is a nuclear medicine imaging technology concerned with measuring the distribution of a particular class of exogenous contrast agents known as radioactive tracers ("radiotracers") inside the body of a patient. The purpose of these measurements is to provide tomographically-reconstructed images to a physician to aid in disease diagnosis or monitoring of a therapy regimen. Radiotracers introduced into the body, by injection, ingestion or inhalation, distribute according to their chemical properties and can be used, for example, to measure cardiac blood flow or to localize tumors. SPECT radiotracers have radioactive atoms that emit gamma-rays detectable by a gamma-ray detector, such as a gamma camera. The gamma-ray detector measures the spatial distribution of gamma-rays that are being emitted by the radiotracer. More specifically, the gamma-ray detector measures the two-dimensional projection of the three dimensional distribution of the radiotracer distribution within the body. From these projections, the three-dimensional distribution of the radiotracer can be reconstructed using the principles of computed tomography.

The intent of SPECT is to produce a cross-sectional image of an entire transaxial slice plane (that is, width) of the patient's body. Indeed, conventional tomographic reconstruction techniques require that each projection span the entire width of the body. These techniques require that, for a given transaxial slice, all of the radiotracer be present within the field-of-view ("FOV") of the gamma-ray detector at the level of that slice. Failure to meet this condition results in truncation of projection data, which in turn, leads to severe artifacts in the tomographically reconstructed images. The study of reconstructing images using truncated data is referred to as local tomography.

An increasing emphasis for SPECT is the need for quantitative accuracy. In SPECT, this is a challenging requirement because gamma-rays emitted from the radiotracer are partially absorbed and scattered by surrounding tissue. As a result, these gamma-rays may not be detected by the gamma-ray detector, or may be mispositioned. Scatter and attenuation generally can only be corrected if the anatomical distribution of surrounding tissue is known.

Gamma-ray detectors are often comprised of a large planar crystal (approximately 30x30cm or larger) coupled to an array of photomultiplier tubes ("PMTs"). The gamma-rays emitted from the radiotracer strike the crystal which converts them into a signal that is detectable by the PMT array. Both the spatial position and the energy of the gamma-rays are thus recorded, subject to the limited spatial and energy resolution imposed by the crystal and PMT array. A promising alternative to imaging using the crystal/PMT array combination is the solid state gamma-ray detector. Examples of solid-state gamma-ray detectors include high purity germanium and cadmium zinc telluride gamma-ray detectors, both of which offer resolution that is superior compared with contemporary crystals, such as sodium iodide and lutetium oxyorthosilicate. Unfortunately, a major disadvantage of solid-state gamma-ray detectors is their high cost, which largely prevents the manufacture of large scale ones. The use of small solid-state gamma-ray detectors with current reconstruction techniques introduces "hot-rim" truncation artifacts that compromise image accuracy when the distribution of activity extends beyond a region-of-interest ("ROI") that is targeted by the FOV of the gamma-ray detector as it rotates about the patient.

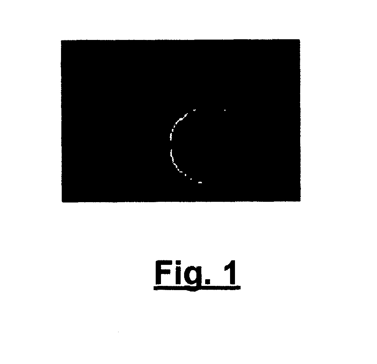

Figure 1 illustrates an image captured using a small gamma-ray detector that clearly demonstrates a "hot-rim" truncation artifact, wherein image data outside the ROI is constrained to the ROI and, thus, is concentrated along the periphery of the ROI.

This imaging size restriction represents a major impediment to using solid-state detectors for tomographic imaging. However, if the impediments associated with reconstruction artifacts could be overcome, then smaller-scale solid-state gamma-ray detectors with their reduced FOV would be preferred for many applications due to their reduced costs.

Imaging using multiple modalities, referred to as hybrid imaging, is known. One such technique of imaging is the use of anatomical imaging with molecular imaging. This hybrid imaging technique permits the detection of various conditions within an object, using the molecular imaging, that are classified based on their location provided by the anatomical imaging.

Recently, several manufacturers have forayed into the emerging area of hybrid imaging, wherein, molecular imaging and anatomical imaging capabilities are brought together into a single system. For example, SPECT and X-ray CT systems have been integrated into a single system referred to as a "SPECT/CT" system. SPECT/CT is intended to overcome the individual limitations of SPECT and CT by offering the visualization of superimposed or "fused" anatomic and molecular information. The X-ray CT images also provide the anatomical information that is required to determine the scatter and attenuation correction necessary for quantitative SPECT. Typically, the X-ray CT images are reconstructed first, and then used to guide the scatter and attenuation correction for the SPECT data.

A number of solutions have been proposed for performing hybrid imaging. For example, the publication entitled "Object specific attenuation correction of SPECT with correlated duat-energy X-ray CT" authored by Hasewaga et al., 1993, IEEE Transactions on Nuclear Science, Volume 40, pp. 1242-1252, discloses the unification of different imaging modalities into a single hybrid imaging platform for SPECT/CT. U.S. Patent No. 6,490,476 to Townsend et al. discloses the same for PET/CT. In both cases, X-ray CT data-guided attenuation is used to correct the gamma-ray measurements.

Several authors have disclosed methods for local tomography, both in SPECT and X-ray CT data. For example, the publication entitled "Local Tomography Property of Residual Minimization Reconstruction With Planar Integral Data" authored by Zeng et al., 2003, IEEE Transactions on Nuclear Science, Volume 50, pp. 1590-1594 describes an iterative algorithm for local SPECT tomography.

U.S. Patent Application Publication No. 2003/0164452 to Van Dulmen et al. discloses a method for local SPECT using a solid-state gamma-ray detector having a FOV that is smaller than the cross-sectional area of the body that relies on a single modality of imaging.

U.S. Patent Application Publication No. 2005/0189494 to Conwell discloses a method for emission and transmission imaging with a small FOV gamma-ray detector. A single gamma-ray detector is used for collecting both emission and transmission data resulting in the truncation of both the emission and transmission data.

U.S. Patent Application Publication No. 2005/0249432 to Zou et al. discloses a method and system for reconstructing a region-of-interest for an object by applying a method of "chords" to truncated data.

Although the above references disclose various methods of imaging objects, improvements in hybrid imaging are desired. It is therefore an object of the present invention to provide a novel method and system for performing local tomography.

### Summary of the Invention

Accordingly, in one aspect there is provided a system for performing local tomography, comprising:
an anatomical imaging subsystem imaging a patient and generating anatomical image data;
a computing device generating an anatomical image from said anatomical image data, and identifying a region-of-interest within said anatomical image; and
a molecular imaging subsystem capturing molecular image data from only a portion of said patient including said region-of-interest, wherein said computing device generates a molecular tomographic image of said region-of-interest from said molecular image data and said anatomical image.

In one embodiment, the molecular imaging subsystem is moved relative to the patient during capturing of the molecular image data to maintain the region-of-interest within the field-of-view thereof The patient and molecular imaging subsystem rotate relative to one another during capturing of the molecular image data and the molecular imaging subsystem is shifted laterally during the relative rotation. As a result, the molecular imaging subsystem follows a generally sinusoidal path. During generation of the tomographic image, the computing device generates an estimated sinugraph representation based on the molecular image data, translates the estimated sinugraph representation into a tomograpic representation, adjusts the tomographic representation to remove extraneous data, projects the adjusted tomographic representation into a sinugraph representation and performs the translating, adjusting and projecting iteratively until the adjusted tomographic representation is sufficient to form the tomographic image.

In one embodiment, the molecular imaging subsystem comprises a gamma-ray detector and the anatomical imaging subsystem comprises a scanner for endogenous contrast detection.

In accordance with another aspect, there is provided a method for performing local tomography, comprising:
receiving anatomical image data of a patient;
receiving molecular image data of a region-of-interest of a patient captured using a detector having a field-of-view that is smaller than said patient; and
generating a local tomographic image of said region-of-interest from said molecular image data using said anatomical image data.

### Brief Description of the Drawings

Embodiments will now be described more fully with reference to the accompanying drawings in which:
Figure 1 is a tomographic image having a hot-rim truncation artifact captured with a small gamma-ray detector;
Figures 2A and 2B illustrate a system for performing local tomography including a small gamma-ray detector;
Figure 3 illustrates the geometry of the small gamma-ray detector of the system of Figures 2A and 2B;
Figure 4 is a schematic representation of a computing device forming part of the system of Figures 2A and 2B;
Figure 5 is a flowchart of the general method for performing local tomography using the system of Figures 2A and 2B;
Figure 6 is a tomographic image of a patient generated using an anatomical imaging subsystem forming part of the system of Figures 2A and 2B;
Figure 7 illustrates the placement of a region-of-interest (ROI) in the tomographic image of Figure 6;
Figures 8A to 8C illustrate the positioning of the gamma-ray detector in order to keep its field-of-view (FOV) focused on the ROI of Figure 7;
Figure 9 illustrates a sinugraphic mask corresponding to the location of the ROI relative to the view range of the gamma-ray detector;
Figure 10 is a flowchart of the method for generating a local tomographic image used by the system of Figures 2A and 2B;
Figures 11 A to 11D illustrate various intermediate sinugraphic and tomographic images generated during the method of Figure 10, and the final generated local tomographic image;
Figures 12A to 12F show tomographic images captured using various hybrid imaging systems; and
Figure 13 illustrates an alternative gamma-ray detector having a prismatic scanning geometry.

### Detailed Description of the Embodiments

A novel medical imaging method and system is presented for generating local tomographic images of the distribution of an exogenous contrast agent in an object using an exogenous contrast agent detector that has a field-of-view (FOV) that is smaller than the object. The method enables the tomographic reconstruction of the exogenous contrast agent within a region-of-interest (ROI) that is the target of the FOV of the exogenous contrast agent detector, while substantially reducing the imaging artifacts associated with a severely truncated dataset. The method relies upon hybrid anatomical imaging / molecular imaging. The method also facilitates the correction of physical effects that would otherwise compromise accurate tomographic reconstruction of the distribution of the exogenous contrast agent, where portions of those physical effects arise from exogenous contrast agent and anatomical image data beyond the ROI targeted by the exogenous contrast agent detector's FOV.

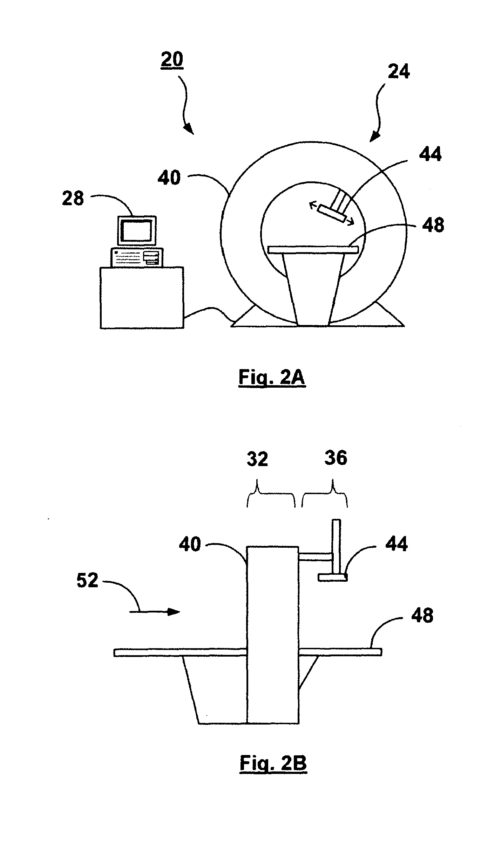

Figures 2A and 2B show a system 20 for performing local tomography. The system 20 includes an imaging apparatus 24 in communication with a computing device 28. The imaging apparatus 24 comprises an anatomical imaging subsystem 32 and a molecular imaging subsystem 36. The anatomical imaging subsystem 32 comprises a rotational X-ray scanner 40 for endogenous contrast detection. The molecular imaging subsystem 36 comprises a gamma-ray detector 44 coupled to the rotational X-ray scanner 40 for exogenous contrast agent detection. The gamma-ray detector 44 has a FOV that is smaller than the patient to be images and may be one of the solid state type gamma-ray detectors referred to previously. A bed 48 is provided for positioning the patient for imaging with the anatomical and molecular imaging subsystems 32, 36. The rotational X-ray scanner 40 and the gamma-ray detector 44 are operable to rotate about the patient to capture image data in a cross-section of the patient. In addition, the gamma-ray detector 44 is movable via a motor along a track that is tangential to the orbit of rotation of the X-ray scanner 40 in order to laterally shift the FOV of the gamma-ray detector 44. The bed 48 is operable to travel longitudinally through the anatomical and molecular imaging subsystems 32, 36 to permit different cross-sections of the patient to be imaged and, of more interest, to permit the same cross-sections of the patient to be imaged by the anatomical imaging subsystem 32 and then by the molecular imaging subsystem 36 by movement of the bed 48 in a forward direction as identified by arrow 52.

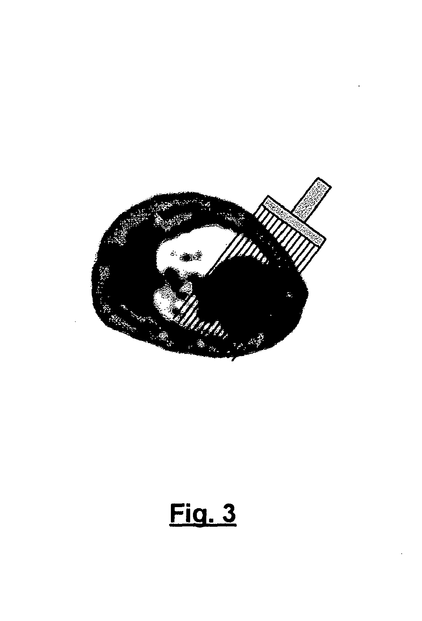

Figure 3 illustrates the parallel geometry of image data capture of the gamma-ray detector 44. As a result of the parallel geometry and the size of the gamma-ray detector 44, the FOV of the gamma-ray detector 44 is not large enough to image the entire patient.

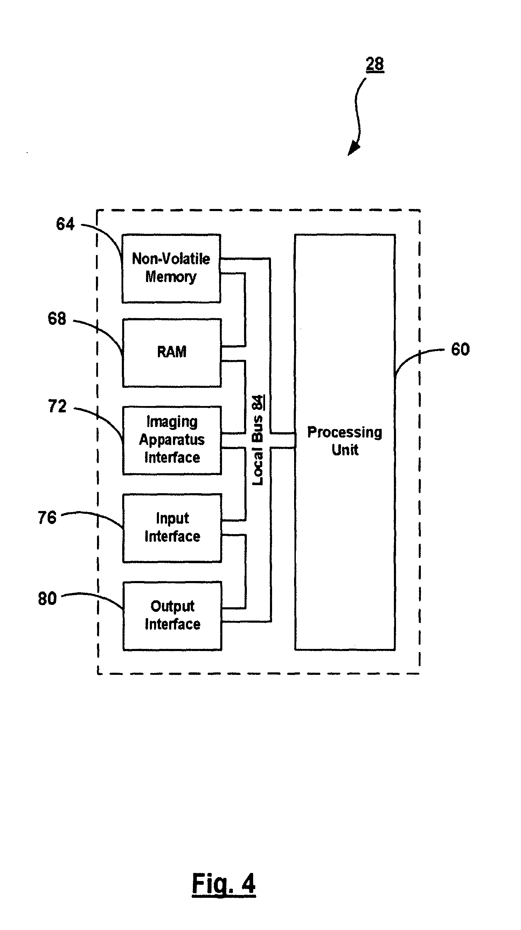

Figure 4 shows various components of the computing device 28 for operating the imaging apparatus 24 and presenting images captured to an operator. The computing device 28 in this embodiment is a personal computer that executes imaging software to enable it to perform local tomography. As can be seen, the computing device 28 comprises a processing unit 60, non-volatile memory 64, random access memory ("RAM") 68, an imaging apparatus interface 72, an input interface 76 and an output interface 80, all in communication over a local bus 84. The processing unit 60 loads and executes software stored in non-volatile memory 64 for performing local tomography. The processing unit 60 registers any ongoing calculations performed during the local tomography in RAM 68. The computing device 28 is coupled to the imaging apparatus 24 via the imaging apparatus interface 72 for sending control commands to the imaging apparatus 24 and for receiving image data therefrom. The control commands direct the operation of the imaging apparatus 24 and the image data includes image data received from both the anatomical imaging subsystem 32 and the molecular imaging subsystem 36. The input interface 76 can include a keyboard, mouse and/or other user input device for interacting with the software, such as for modifying various settings/thresholds, etc. The output interface 80 includes a display for visually presenting the results of the local tomography, if so desired, and can display settings of the software to allow for their adjustment.

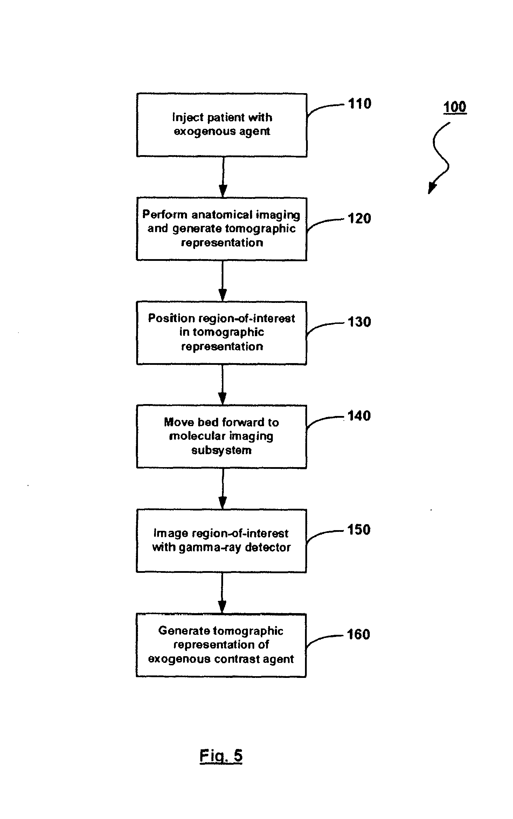

The method 100 for performing local tomography via the system 20 will now be described more fully with reference to Figure 5. Initially, the patient to be imaged is injected with an exogenous contrast agent and placed on the bed 48 of the system 20 (step 110). Anatomical imaging is then performed on the patient and a tomographic representation or image is generated (step 120). The anatomical image data collected by the anatomical imaging subsystem 32 is reconstructed to yield a tomographic representation (that is, a cross-sectional image) of the patient's anatomy.

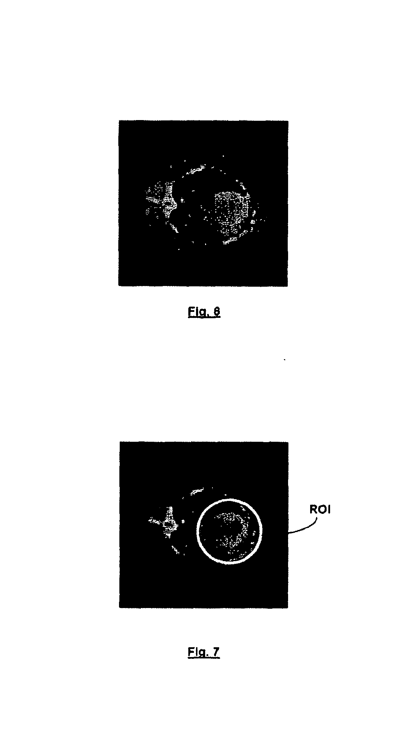

Figure 6 shows such a tomographic representation of the patient imaged by the anatomical imaging subsystem 32 and generated by the computing device 28. The patient, in this case, is a canine and the cross-section shown is taken of the canine's thorax.

With the tomographic image generated by the anatomical imaging subsystem 32 displayed, an operator positions the target ROI of the molecular imaging subsystem 36 on the tomographic image using the mouse and/or keyboard of the computing device 28 (step 130). Figure 7 shows the tomographic image of Figure 6 with the ROI overlaid thereon.

The bed 48 is then moved longitudinally to position the patient such that the area of the patient's body, including the ROI to be imaged, is within the cross-section viewed by the molecular imaging subsystem 36 (step 140). The ROI determines the physical placement of the gamma-ray detector 44 for imaging. The ROI is then imaged as the X-ray scanner 40 and hence, gamma-ray detector 44 rotates about the patient (step 150).

As the gamma-ray detector 44 rotates about the patient, the location of the target volume identified by the ROI positioned on the tomographic image can exit the FOV of the gamma-ray detector 44 if the ROI is not centered in the circular path of the rotational X-ray scanner 40 to which the gamma-ray detector 44 is coupled. As a result, during rotation of the X-ray scanner 40, the position of the gamma-ray detector 44 is shifted laterally in order to maintain the ROI within the FOV of the gamma-ray detector 44.

Figures 8A to 8C illustrate the capture of image data by the gamma-ray detector 44 as it rotates about the patient. As shown, the gamma-ray detector 44 is shifted laterally along a track secured to the rotational X-ray scanner 40 during rotation such that the FOV of the gamma-ray detector 44 remains focused on the ROI. The entire range of potential FOVs is referred to as a view range.

In Figure 8A, the gamma-ray detector 44 is shown in a first position, referred to as position A. The gamma-ray detector 44 is positioned slightly above three o'clock and is shifted laterally clockwise within the view range so that the ROI is entirely within the FOV of the gamma-ray detector 44. Note that the FOV of the gamma-ray detector 44 also captures image data from other areas of the patient outside the ROI. This image data is deemed noise and dealt with in a manner described hereinbelow.

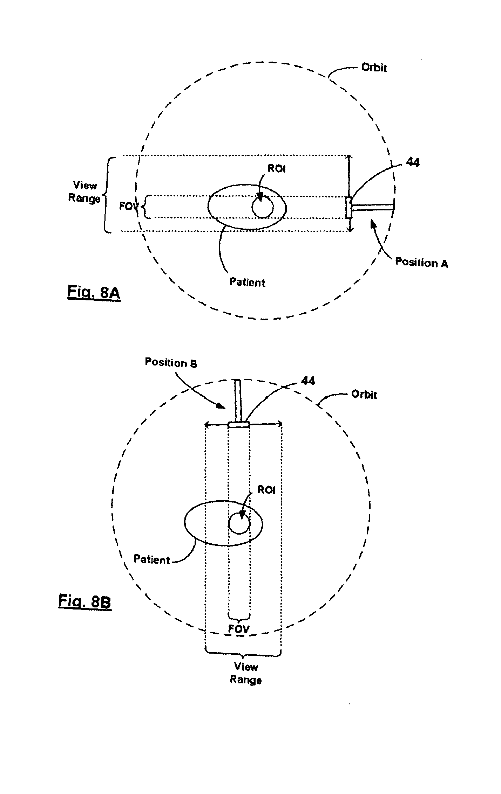

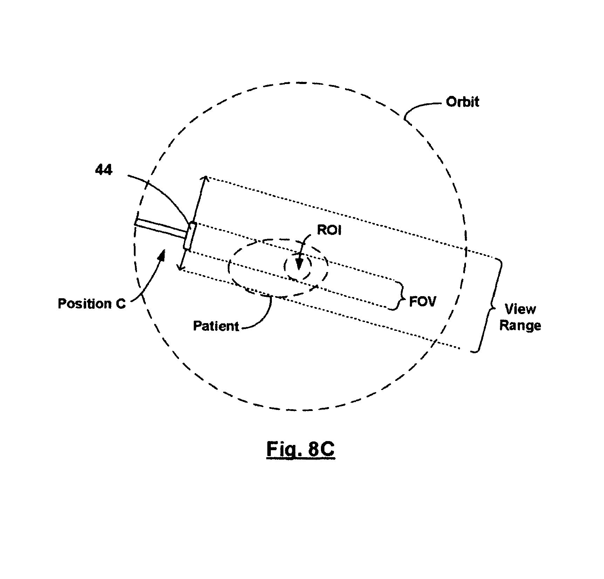

Figure 8B shows the gamma-ray detector 44 in a second position, referred to as position B, wherein it is rotated to a position slightly to the left of twelve o'clock, and shifted slightly laterally in the counter-clockwise direction within the view range of the gamma-ray detector 44.

Figure 8C shows the gamma-ray detector 44 in a third position, referred to as position C, wherein it is rotated to a position slightly above nine o'clock, and shifted laterally in the counter-clockwise direction within the view range of the gamma-ray detector 44.

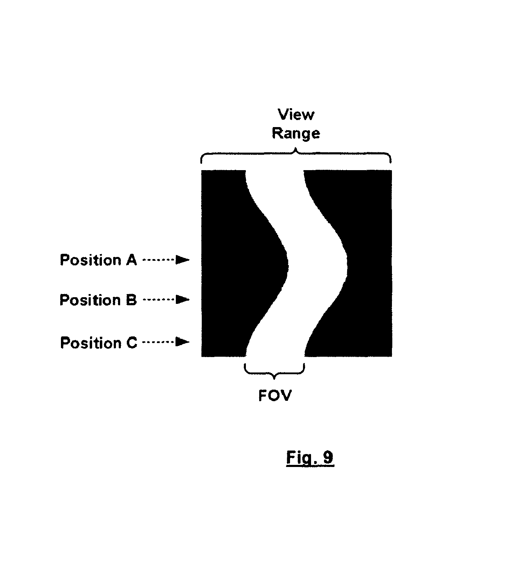

The lateral shifting of the gamma-ray detector 44 within the view range during rotation about the patient to keep the FOV focused on the ROI defines a binary mask as shown in Figure 9. The binary mask is a sinugraphic representation of the lateral shifting of the gamma-ray detector 44 in order to keep the ROI within the FOV of the gamma-ray detector 44 as it rotates about the patient. The lateral shift of the gamma-ray detector 44 at position A, position B and position C are clearly labeled on the binary mask. As can be seen, at position A, the FOV of the gamma-ray detector 44 is shifted to the right of center (that is, clockwise). At position B, the FOV of the gamma-ray detector 44 is shifted slightly left of center (that is, counter-clockwise). At position C, the FOV of the gamma-ray detector 44 is shifted left of center (that is, counter-clockwise).

As mentioned above, the ROI is imaged using the gamma-ray detector 44 as the gamma-ray detector 44 is shifted laterally in accordance with the binary mask to maintain the ROI within the FOV of the gamma-ray detector. A tomographic representation of the exogenous contrast agent is then generated from the image data captured by the gamma-ray detector 44 (step 160).

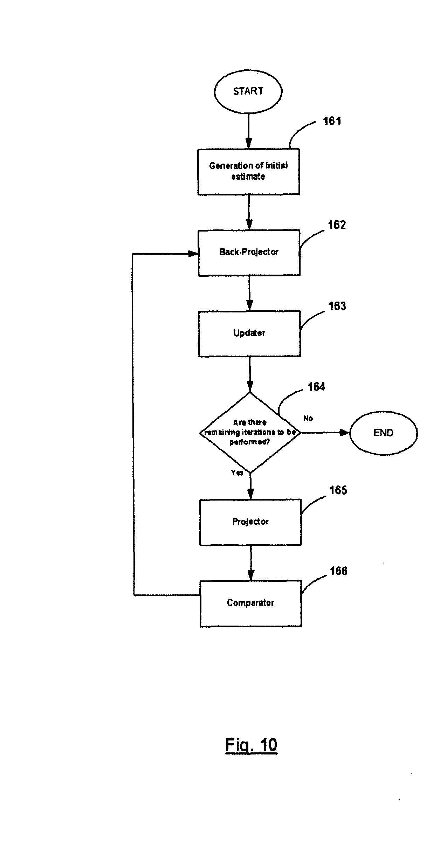

Figure 10 is a flowchart of the steps performed during generation of the tomographic representation of the exogenous contrast agent. The method for reconstruction is iterative, deriving from the work on maximum likelihood expectation maximization ("MLEM") disclosed in the publication entitled "EM Reconstruction algorithms for emission and transmission tomography" authored by Lange et al., Computer Assisted Tomography, Volume 8, pp. 308-316, and its accelerated derivatives, such as ordered subsets expectation maximization as disclosed in the publication entitled "Accelerated image reconstruction using ordered subsets of projection data" authored by Hudson et al., 1994, IEEE Transactions on Medical Imaging, Volume 13, pp. 601-609. MLEM algorithms allow for the modeling of various physical phenomena such as photon propagation through complex media and incorporate statistical aspects of signal detection. In accordance with most other MLEM-type algorithms, the iterative method employed by the system 20 is comprised of four main component steps as will be explained.

During the method of generating the tomographic representation of the exogenous contrast agent at step 160, an initial estimate of the sinugraphic representation is generated (step 161). The initial estimate is generated by performing a cross-sectional scan with the gamma-ray detector 44 and preparing a sinugraphic representation of the image data. In performing the cross-sectional scan, the gamma-ray detector 44 captures image data along a cross-sectional plane of the patient. The image data is captured along a single pixel row that is aligned with the plane.

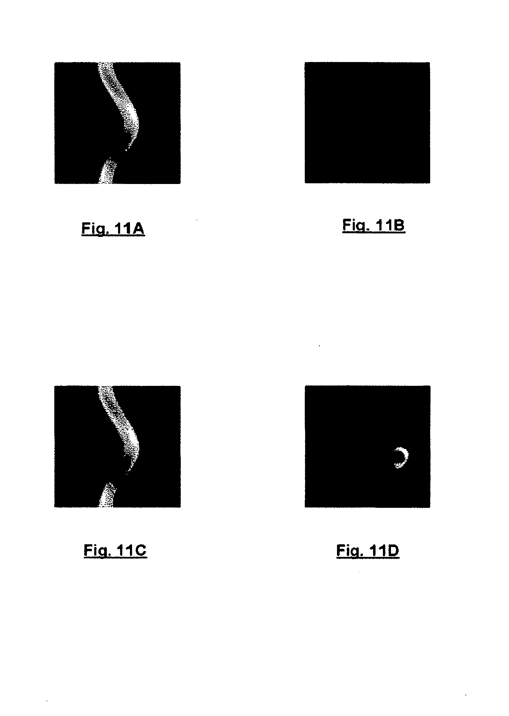

Figure 11A shows a sinugraphic representation of the exogenous contrast agent image data. Each pixel row of the sinugraphic representation of the image data is captured at a different position as the gamma-ray detector 44 rotates about the patient and is offset to reflect the lateral shift of the gamma-ray detector 44. As can be seen, the sinugraphic representation appears to include some noise. The noise is the result of exogenous contrast agent detected by the gamma-ray detector 44 as it rotates about the patient and captures image data that has settled in other areas of the patient outside of the ROI. This is best illustrated by referring again to Figure 8A, where the FOV of the gamma-ray detector 44 captures image data both inside the ROI and outside the ROI, such as to the left of the ROI in the patient. As some of the exogenous contrast agent may have settled in other areas of the patient, this exogenous contrast agent may appear in the image data collected by the gamma-ray detector at imaging angles that pass through these other areas of the patient.

Next, a back-projector is used to transform the sinugraphic representation generated at step 161 into a tomographic representation (step 162). The back-projector operates to re-construct a two-dimensional cross-sectional representation of the exogenous contrast agent image data. During this reconstruction, the exogenous contrast agent image data that appears to be noise is assumed to be from outside the ROI in the patient and is represented in the tomographic image outside the ROI in accordance with a best estimate of its location prepared by the back-projector.

Figure 11 B illustrates a tomographic representation generated from the sinugraphic representation of Figure 11A. As can be seen, the tomographic image is not as clear as desired. Further, while not clearly visible, exogenous contrast agent residing in the patient to the left of the main concentration in the ROI appears in the image.

Once the tomographic image has been generated, an updater adjusts the tomographic image by limiting exogenous contrast agent image data to the area of the patient delineated from the anatomical image (step 163). A binary mask is generated from the anatomical image of the patient as shown in Figure 6, with regions inside the patient being set to one and areas outside the patient being set to zero. This mask is applied to the tomographic image generated at step 162 to remove exogenous contrast agent image data estimated to be outside the patient's body.

If the number of iterations of the method specified by the operator have been performed at this point, processing of the exogenous contrast agent image data ceases and the generated tomographic image is output (step 164).

If, instead, further iterations remain to be performed, upon adjusting the tomographic image, a projector transforms the tomographic representation adjusted at step 163 into a sinugraphic representation (step 165). The sinugraphic representation is generated by estimating the exogenous contrast agent image data that would be collected by the gamma-ray detector 44 were the exogenous contrast agent to be distributed in the patient in accordance with the adjusted tomographic image generated at step 163.

Figure 11C shows a sinugraphic representation reconstructed from the tomographic image of Figure 11B. As can be seen, the sinugraphic representation illustrates a main swath of image data that corresponds generally to the shape of the binary mask of Figure 9, plus out-of-phase image data that corresponds to exogenous contrast agent that is captured by the gamma-ray detector 44 as its FOV captures image data from other areas of the patient.

Once the sinugraphic representation is re-constructed, a comparator separates what is deemed to be image data from the ROI from image data that is not deemed to be from the ROI (step 166). In order to distinguish image data deemed to be from the ROI from that image data that is deemed to be from outside the ROI, the comparator examines the image data in the sinugraphic representation that corresponds to the binary mask of Figure 9, estimates what image data corresponds to noise and removes the image data deemed to be noise from the sinugraphic image. The removed image data can include image data within the white swath of the binary mask that is deemed to be noise.

Once the sinugraphic representation has been filtered in this manner by the comparator at step 166, it is then processed by the back-projector again to re-generate a tomographic representation at step 162.

The method of generating a tomographic representation operates in a loop, where the number of iterations is specified by the operator. As will be understood, during each iteration, a portion of the noise is eliminated, resulting in a sharper image. Increasing the number of iterations, however, provides for a more accurate reconstruction but is more time consuming.

Figure 11D illustrates the final tomographic image generated by the system 20 after multiple iterations. As can be seen, the image is sufficiently clear to distinguish features therein.

### TESTING

Figures 12A to 12F show quantitative local cardiac tomography and washout kinetic modeling over 24 hours in a 23kg canine with three tracers having very different distributions, using data from as little as an 11 × 11cm FOV. Bone marrow mesenchymal cells were transfected with a reporter gene, radio-labeled with In-111 and transplanted into myocardium, and an I-131-radiolabeled reporter probe was injected peripherally. Tc-99m-Sestamibi was injected to measure perfusion. SPECT/CT was acquired hourly with gamma cameras using different FOVs.

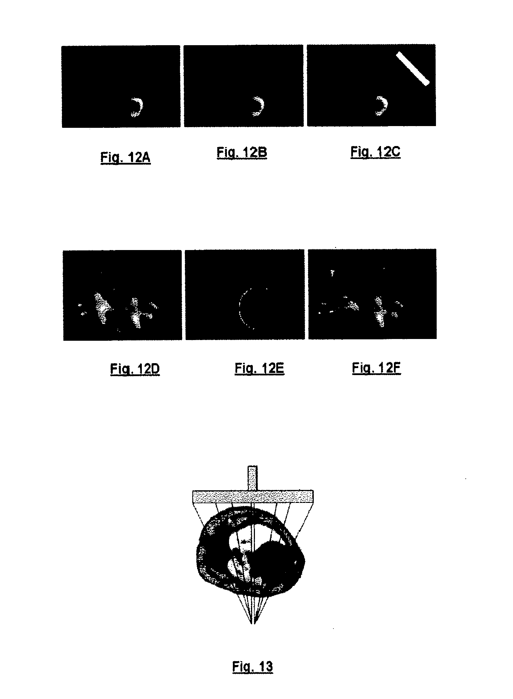

Figures 12A and 12D are images captured with a 34×34cm standard OSEM gamma camera. As can be seen, image quality is good, but the cost of this size of gamma camera is prohibitive. Figures 12B and 12E are images captured with an 11 ×11cm standard OSEM gamma camera. As can be seen in Figure 12E, a "hot rim" artifact appears as all image data captured outside of the ROI is relocated to within the ROI along the periphery thereof. Figures 12C and 12F show images captured using a modified OSEM in accordance with the above-described embodiment. As can be seen in Figure 12F, the system 20 performs local tomography within the ROI well. The system 20 relaxes the constraint that all image data is assumed to come from the ROI by using object boundary definition from non-truncated CT. Using the truncated 11×11cm standard OSEM gamma-ray detector, cardiac bull's-eye analysis revealed errors as large as 12%, whereas in the system 20, errors were about 1%.

The system 20 reduced errors associated with extreme truncation, enabling quantitative local tomography using the small FOV gamma-ray detector 44 when full FOV CT is available.

While, in the above-described embodiment, SPECT and CT are employed, those skilled in the art will recognize that SPECT is only one of many modalities capable of performing molecular imaging, and X-ray CT only is only one of many modalities capable of performing anatomical imaging.

While the above-described embodiment has been described with respect to a gamma-ray detector with a parallel geometry, those skilled in the art will appreciate that other types of detector geometries can be used. For example, Figure 13 illustrates another embodiment wherein the gamma-ray detector is large in spatial extent, but is equipped with a lens or collimator of converging geometry restricting measurements to the ROI defined by the operator.

The bed of the system itself may be fully stationary, and the detectors themselves can move relative to the patient to perform image data capture. The detectors may be located to capture image data from the same plane without motion of the detectors relative to the patient.

The anatomical imaging subsystem and molecular imaging subsystem may use the same detector material and may move in synchrony about the patient, or there may be no detector bed motion, as in the case of hybrid PET/magnetic resonance imaging.

As will be appreciated, in prior art imaging methods, all exogenous contrast is reconstructed within the ROI, resulting in the aforementioned imaging artifacts. In the subject method disclosed herein, the exogenous contrast agent is constrained to be within the boundary of the object, where this boundary is measured using anatomical imaging. During iterative reconstruction, molecular image data projected onto the mask is considered unmeasured and no discrepancy is back-projected.

The imaging software application executed by the computing device may run as a stand-alone medical image processing tool or may be incorporated into other available medical image processing applications to provide enhanced functionality to those medical image processing applications. The software application may include program modules including routines, programs, object components, data structures etc. and be embodied as computer-readable program code stored on a computer-readable medium. The computer-readable medium is any data storage device that can store data, which can thereafter be read by a computer system. Examples of computer-readable media include for example read-only memory, random-access memory, hard disk drives, magnetic tape, CD-ROMs and other optical data storage devices. The computer-readable program code can also be distributed over a network including coupled computer systems so that the computer-readable program code is stored and executed in a distributed fashion.

Although particular embodiments have been described, those of skill in the art will appreciate that variations and modifications may be made without departing from the spirit and scope thereof as defined by the appended claims.

## Claims

1. A system for performing local tomography, comprising:
an anatomical imaging subsystem imaging a patient and generating anatomical image data;
a computing device generating an anatomical image from said anatomical image data, and identifying a region-of-interest within said anatomical image; and
a molecular imaging subsystem capturing molecular image data from only a portion of said patient including said region-of-interest, wherein said computing device generates a molecular tomographic image of said region-of-interest from said molecular image data and said anatomical image.

2. A system according to claim 1 wherein said molecular imaging subsystem is moved relative to said patient during capturing of said molecular image data to maintain the region-of-interest within the field-of-view thereof.

3. A system according to claim 2 wherein said patient and molecular imaging subsystem rotate relative to one another during capturing of said molecular image data

4. A system according to claim 2 wherein said molecular imaging subsystem is shifted laterally during relative rotation between said patient and said molecular imaging subsystem.

5. A system according to claim 1 wherein said anatomical imaging subsystem rotates about said patient during imaging of said patient.

6. A system according to claim 5 wherein said molecular imaging subsystem is coupled to said anatomical imaging subsystem and rotates with said molecular imaging subsystem, said molecular imaging subsystem being shifted during rotation to maintain the region-of-interest within the field-of-view thereof.

7. A system according to claim 6 wherein said molecular imaging subsystem follows a generally sinusoidal path as a result of said rotating and shifting.

8. A system according to any one of claims 1 to 7 wherein said molecular imaging subsystem comprises a gamma-ray detector.

9. A system according to claim 8 wherein said gamma-ray detector is a solid state gamma-ray detector.

10. A system according to any one of claims 1 to 9 wherein said anatomical imaging subsystem comprises a scanner for endogenous contrast detection.

11. A system according to any one of claims 1 to 10 wherein said region-of interest is operator selected.

12. A system according to claim 7 wherein during generation of said tomographic image, said computing device generates an estimated sinugraph representation based on said molecular image data, translates the estimated sinugraph representation into a tomographic representation, adjusts the tomographic representation to remove extraneous data, projects the adjusted tomographic representation into a sinugraph representation and performs the translating, adjusting and projecting iteratively until the adjusted tomographic representation is sufficient to form said tomographic image.

13. A method of performing local tomography, comprising:
receiving anatomical image data of a patient;
receiving molecular image data of a region-of-interest of said patient captured using a detector having a field-of-view that is smaller than said patient; and
generating a local tomographic image of said region-of-interest from said molecular image data using said anatomical image data.

14. The method of claim 13 further comprising capturing said molecular image data by moving said detector relative to said patient and maintaining the region-of interest within the field-of-view of said detector.

15. The method of claim 14 wherein during said moving said detector follows a generally sinusoidal path.

16. The method of claim 15 wherein said tomographic image generating comprises generating an estimated sinugraph representation based on said molecular image data, translating the estimated sinugraph representation into a tomographic representation, adjusting the tomographic representation to remove extraneous data, projecting the adjusted tomographic representation into a sinugraph representation and performing the translating, adjusting and projecting iteratively until the adjusted tomographic representation is sufficient to form said tomographic image.
